# EUROPEAN PATENT SPECIFICATION

(11) **EP 0 867 434 B1**
(45) Date of publication and mention of the grant of the patent: **14.08.2002**
(21) Application number: 98300628.9
(22) Date of filing: 29.01.1998
(51) Int. Cl.: C07D 317/12

(54) **A method for continuously producing a cyclic formal**
Ein Verfahren zur kontinuierlichen Herstellung eines cyclischen Formals
Un procédé pour la préparation d'un formal cyclique

(30) Priority: 29.01.1997 JP 1522897; 07.08.1997 JP 21375397
(43) Date of publication of application: 30.09.1998
(73) Proprietor: TORAY INDUSTRIES, INC., Tokyo 103 (JP)
(72) Inventor: Ogawa, Daisuke, Midori-ku, Nagoya-shi, Aichi 458-0846 (JP); Koike, Masahiro, Nagoya-shi, Aichi 457-0866 (JP); Yamamoto, Yoshiyuki, Mie 513-0032 (JP)
(74) Representative: Coleiro, Raymond

(56) References cited:
- US-A- 5 456 805
- DATABASE WPI Week 9610 Derwent Publications Ltd., London, GB; AN 96-096244 XP002064240 A.L. BALASHOV ET AL.: "1,3-Dioxolane synthesis method - reacting ethylene glycol and formaldehyde continuously in presence of acid catalyst, using azeotropic distillation and reagent-prod. counterflow" & RU 2 036 919 C (UNIV NIZHNYI NOVGOROD TECH) , 1 March 1993
- DATABASE WPI Week 9346 Derwent Publications Ltd., London, GB; AN 93-365215 XP002064241 "Prepn. of 1,3-di-oxolane for solvents, acetal resin, medical intermediates - by reacting formaldehyde or formaldehyde forming cpd. with ethylene glycol in presence of acid catalyst, evaporating resultant reaction mixt. etc." & JP 05 271 217 A (ASAHI CHEM IND CO LTD) , 19 October 1993
- DATABASE WPI Week 9542 Derwent Publications Ltd., London, GB; AN 95-325537 XP002064242 "High purity cyclic formal prepn. - by maintaining molar ratio of alkylene glycol to formaldehyde in presence of catalyst" & JP 07 224 055 A (POLYPLASTICS KK) , 22 August 1995

## Description

The present invention relates to a method for continuously producing a cyclic formal used, for example, as an intermediate product for solvents and drugs or as a raw material in acetal resins. In more detail, the present invention relates to a method for continuously producing a cyclic formal less in the content of impurities such as unreacted formaldehyde and water, by letting an alkylene glycol and formaldehyde continuously react with each other in the presence of a catalyst.

It is known that cyclic formals such as ethylene glycol formal, diethylene glycol formal and 1,4-butanediol formal can be obtained by cyclic reaction between a corresponding alkylene glycol or alkylene oxide and aldehyde. For example, DE-C-0191420 discloses a method for producing ethylene glycol formal, a typical cyclic formal, by letting ethylene glycol formal and formaldehyde react with each other in the presence of an acid catalyst.

Furthermore, JP-A-49-062469 discloses a method for producing ethylene glycol formal by letting ethylene glycol and paraformaldehyde react with each other in the presence of an acid catalyst.

Moreover, JP-A-07-224055 discloses a method for obtaining a cyclic formal by letting an alkylene glycol and formaldehyde react with each other in a specific molar ratio.

The cyclic formal obtained by any of the above methods is usually obtained as a mixture consisting of a cyclic formal containing a large amount of water, and free water. Methods for decreasing water from the mixture containing water, for obtaining a purified cyclic formal are also known. For example, JP-A-49-062469 discloses a method for producing a purified cyclic formal by adding cyclohexane to the reaction distillate obtained by letting paraformaldehyde and ethylene glycol react with each other in the presence of an acid catalyst, and rectifying the mixture. JP-A-03-024071 discloses a purification method in which a crude cyclic acetal is treated with an alkali aqueous solution and/or solid alkali hydroxide compound. JP-A-05-271217 discloses a purification method in which the reaction distillate obtained by letting formaldehyde and ethylene glycol react with each other in the presence of an acid catalyst is brought into contact with ethylene glycol in counter current in a distillation tower. JP-A-07-206716 discloses a purification method in which a mixture containing a cyclic formal and water is brought into contact with a hydrophilic solvent with a boiling point of 180 to 250°C in a distillation tower. Furthermore, JP-A-07-206717 discloses a method in which the purified cyclic formal obtained by the process of JP-A-07-206716 is further treated with a dehydrating agent. In addition, US 5456805 discloses a purification process characterised by supplying a mixture of cyclic formal and water into a distillation tower, effecting distillation while supplying n-pentane into the tower and taking out a purified cyclic formal as a bottom liquid.

However, the conventional methods for producing a cyclic formal still have practical problems to be solved. For example, if a cyclic formal is synthesized by letting an alkylene glycol and formaldehyde react with each other, and the product containing the cyclic formal is taken out of the reaction mixture, then the product still contains unreacted formaldehyde. For this reason, these methods do not yet reach a practically satisfactory level as methods for producing a cyclic formal. The formaldehyde present in the cyclic formal affects the synthesis process and the subsequent purification process. For example, formaldehyde is converted into formic acid by Cannizzaro's reaction, to pose such problems that the equipment is corroded and that the entire equipment is complicated.

Because of these problems, in the production of a cyclic formal, it is desired to obtain a cyclic formal not containing unreacted formaldehyde as far as possible.

The conventional methods for purifying a cyclic formal also have practical problems to be solved. For example, in the method of adding cyclohexane to a cyclic formal and rectifying the mixture, the decrease in water content is not sufficient. Furthermore, in the method of bringing a cyclic formal into contact with ethylene glycol or a hydrophilic solvent with a boiling point of 180 to 250°C for distillation, although the water content of the cyclic formal decreases, water migrates into ethylene glycol or hydrophilic solvent, to require further purification of the ethylene glycol or solvent. Therefore, the number of steps increases to complicate the process disadvantageously, and problems are not solved to a practically satisfactory level.

Because of the above situation, a process for producing a cyclic formal less in the content of impurities, consistent from the synthesis to purification of the cyclic formal, is desired.

We studied intensively a method for continuously producing a cyclic formal with a large water content but with a small unreacted formaldehyde content by letting an alkylene glycol and formaldehyde react with each other, and then distilling the cyclic formal with a large water content for continuously purifying it. As a result, we found that a cyclic formal with a small unreacted formaldehyde content can be easily continuously produced by continuously supplying a raw alkylene glycol to a distillation tower when unreacted formaldehyde and a cyclic formal are distilled out, and on the other hand, by continuously supplying raw formaldehyde to a reactor.

Furthermore, we found that a cyclic formal small in the content of water and other impurities can be easily continuously obtained by distilling the water-containing cyclic formal for continuously obtaining an azeotropic mixture consisting of the cyclic formal and water, and adding a specific hydrocarbon compound capable of forming another azeotropic mixture with water, to the azeotropic mixture consisting of the cyclic formal and water, for continuous distillation.

The present invention addresses the problem of providing a method for continuously producing a cyclic formal with a small impurities content by a process consistent from the synthesis and purification of the cyclic formal.

The present invention also addresses the problem of providing a method for producing a cyclic formal with small aldehyde and water contents and of providing an easy and simple method for continuously producing a purified cyclic formal small in the content of water and other impurities and highly improved in rectification efficiency.

The accompanying Figs. 1 and 2 are schematic process drawings for illustrating example cyclic formal production processes. Figs. 3 and 4 illustrate example purification processes.

The present invention provides a method for continuously producing a cyclic formal, in which a reaction distillation tower equipped with a reactor and a distillation tower is used for letting an alkylene glycol component and a formaldehyde component react with each other in the presence of a catalyst, to produce a cyclic formal, comprising the step of continuously supplying the alkylene glycol component from the distillation tower to the reactor of the reaction distillation tower, while continuously supplying formaldehyde component to the reactor of the reaction distillation tower, for letting both the alkylene glycol and formaldehyde components react with each other.

The present invention also provides a method for continuously producing a cyclic formal, in which the reaction distillation tower is used to let an alkylene glycol component and a formaldehyde component react with each other in the presence of a catalyst, to produce a cyclic formal, comprising
(1) the step of continuously supplying an alkylene glycol component from the distillation tower to the reactor of the reaction distillation tower while continuously supplying formaldehyde component to the reactor of the reaction distillation tower, for letting both the alkylene glycol and formaldehyde component react with each other, to continuously obtain a reaction distillate containing water and a cyclic formal from an upper station of the distillation tower,
(2) the step of continuously supplying the reaction distillate containing water and a cyclic formal obtained in step (1) to a second distillation tower, to continuously obtain an azeotropic mixture consisting of the cyclic formal and water, and
(3) the step of supplying the azeotropic mixture consisting of the cyclic formal and water obtained in step (2) to an azeotropic distillation tower, while supplying a hydrocarbon component, the azeotropic point of which with water is not higher than the boiling point of the cyclic formal, for continuous distillation, and obtaining the azeotropic mixture consisting of the hydrocarbon component and water with the cyclic formal from an upper station of the azeotropic distillation tower while continuously obtaining the cyclic formal purified after removing water, from an known station of the tower.

Preferred embodiments of the present invention include the following:
a. The alkylene glycol component supplied to the distillation tower is brought into contact with the unreacted formaldehyde coming from the reactor in counter current, to absorb or react with the formaldehyde component, while being introduced into the reactor.
b. While the molar ratio of the alkylene glycol component to formaldehyde component in the reactor is kept at 1 or more, the amounts of the alkylene glycol and formaldehyde components continuously supplied to the distillation tower and the reactor are kept practically equimolar.
c. The alkylene glycol component is at least one alkylene glycol selected from ethylene glycol, diethylene glycol and 1,4-butanediol.
d. The reaction distillation tower used has two or more distillation stations in the distillation tower.
e. The distillation tower of the reaction distillation tower is a plate tower and/or a packed tower.
f. In step (3), the hydrocarbon component is at least one hydrocarbon compound selected from cyclopentane, cyclohexene and benzene.
g. In step (3), the azeotropic mixture consisting of a cyclic formal and water is supplied to an upper station or a reflux portion (which is usually at an upper station) of the azeotropic distillation tower.
h. In said step (3), the distillate from the azeotropic distillation tower is at least partially refluxed to the azeotropic distillation tower, without discharging all of the quantity of the distillate outside the system.
   Embodiments of the present invention also include a method for continuously purifying a cyclic formal, in which water is removed from a water-containing cyclic formal by distillation, comprising the step of adding a hydrocarbon component which is at least one hydrocarbon solvent selected from cyclopentane, cyclohexene and benzene and the azeotropic point of which with water is not higher than the boiling point of the cyclic formal, to the water-containing cyclic formal, for continuous distillation. Preferred embodiments of this continuous purification method includes the following.
i. The cyclic formal is at least one formal selected from ethylene glycol formal, diethylene glycol formal and 1,4-butanediol formal.
j. The water-containing cyclic formal is supplied to an upper station or a reflux portion (which is usually at an upper station) of the distillation tower.
k. The distillate of the distillation tower is at least partially refluxed to the distillation tower, without discharging all the quantity of the distillate outside the system.

Embodiments of the present invention are described below in detail.

The alkylene glycols which can be used in the present invention include ethylene glycol, diethylene glycol, 1,2-propanediol, 1,3-propanediol, 1,4-butanediol. 1,5-pentanediol, 1,6-hexanediol and (hydromethoxy)ethanol. Among them, ethylene glycol, diethylene glycol and 1,4-butanediol are preferable since they are high in industrial utilization value. Ethylene glycol is especially preferable.

The formaldehyde component as the other raw material of the reaction can be, for example, formaldehyde gas, formaldehyde aqueous solution or paraformaldehyde. In view of easy handling, the use of formaldehyde aqueous solution is preferable.

As the catalyst used in the present invention, an acid catalyst is suitable. The catalysts which can be used include mineral acids such as sulfuric acid and phosphoric acid, strong organic acids such as sulfonic acid, phosphonic acid and trifluoroacetic acid, solid acid catalysts such as strong acid cation exchange resins, zeolite, silica•alumina and activated clay, and heteropoly-acids such as phosphorus molybdic acid and phosphorus tungstic acid. As the catalyst, an acid nonvolatile under the reaction condition is preferably used.

As the reaction distillation tower used in the step (1) of the present invention, any ordinary known reaction distillation tower can be used. For example, a reaction distillation tower with a distillation tower directly installed in a reactor can be used. Furthermore, a reaction distillation tower in which the distillate from a reactor installed separately from a distillation tower is supplied to a lower station of the distillation tower by a pipe, etc. while the condensate at a lower station of the distillation tower is returned to the reactor by a pipe, etc. can also be used.

The temperature control means for heating and cooling the reactor and the distillation tower of the reaction distillation tower can be any ordinary means such as steam, heating medium or electric heating, for example.

In the present invention, the distillation tower of the reaction distillation tower can be, for example, a plate tower or packed tower.

The plate type of the plate tower can be any type such as bubble cap, sieve tray, Uniflux tray, Flexi tray, Natta float bubble tray, ballast tray, cascade tray, venturi tray, Kitter tray, recycling tray, chimney tray, Z tray, turbo grid tray, ripple tray, dual flow tray or baffle tray, for example.

The packing of the packed tower can be of any type such as a ring type, saddle type, Dixon ring, Magmahon packing, spray pack, Pana pack, Goodroy packing, Stedman packing or cross spiral packing.

It is preferable that the number of distillation stations in the distillation tower of the reaction distillation tower is 2 or more. Simple distillation is not preferable since the distilled cyclic formal contains much unreactive formaldehyde. If the number of distillation stations of the distillation tower is sufficiently large, operation can be effected without refluxing the condensate coming from an upper station of the distillation tower.

At an upper station of the distillation tower, a condenser can also be installed. If the condenser is used to partially reflux the distillate, the distillation tower can be optimized. Of course, in the present invention, as described above, the cyclic formal can also be produced without refluxing the distillate.

In the execution of the present invention, in step (1), the positions and timings for supplying the alkylene glycol and formaldehyde components as raw materials to the reaction distillation tower are important. The alkylene glycol is supplied to the distillation tower of the reaction distillation tower and brought into contact with unreacted formaldehyde in counter current, for absorbing or reacting with the formaldehyde, to be added to the reactor. On the other hand, it is important that formaldehyde is directly supplied to the reactor for counter current contact of unreactive formaldehyde with the alkylene glycol. In this case, it is preferable that the position at which the alkylene glycol is added to the distillation tower is a middle or higher station of the distillation tower, to enhance the effect of counter current contact with unreacted formaldehyde. However, the position is not limited to a middle or higher station, as far as a cyclic formal with a small unreacted formaldehyde content of, say, 1 wt% or less, preferably 0.5 wt% or less can be obtained as intended in step (1).

In step (1), it is preferable that the molar ratio of the alkylene glycol to formaldehyde in the reactor is 1 or more, and more preferable is 2 or more. A molar ratio of less than 1 is not preferable, since the unreacted formaldehyde content in the distilled cyclic formal is too large. On the other hand, if the molar ratio of the alkylene glycol to formaldehyde in the reactor is larger, a larger reactor is necessary. Therefore, industrially, it is preferable that the molar ratio of the alkylene glycol to formaldehyde is 100 or less.

As for the amounts of the alkylene glycol and formaldehyde supplied to the distillation tower and the reactor in the steady continuous operation state, since they are consumed in equimolar amounts, it is preferable to supply substantially equimolar amounts of them.

For reaction between the alkylene glycol and formaldehyde, ordinary publicly known reaction conditions can be adopted. For example, in the case of ethylene glycol formal, it is preferable that the reaction temperature is 50 to 170°C, and a more preferable range is 80 to 140°C. Furthermore, it is preferable that the reaction pressure is atmospheric pressure to a pressure at which an equimolar amount of water produced in the ethylene glycol formal reaction and the water contained in the raw formalin can be evaporated.

The vapor obtained in this reaction contains a cyclic formal, water and unreacted formaldehyde. Especially as water, the reaction water equal in moles to the cyclic formal and the water in the formalin are distilled together. Thus, in step (1), the cyclic formal is obtained as a mixture with the substantially equimolar amount of water produced in the reaction and the water in the raw formalin (reaction distillate).

Then, the obtained reaction distillate is distilled, but since the cyclic formal and water form an azeotropic mixture, it is difficult to sufficiently separate and remove water by ordinary distillation only. So, the obtained mixture of the cyclic formal and water is transferred to step (2) wherein the azeotropic mixture consisting of the cyclic formal and water is obtained.

The second distillation tower used in step (2) can be an ordinary known distillation tower. As in the case of the distillation tower of step (1), if a plate tower is used, the plate type can be any type such as a bubble cap, sieve tray, Uniflux tray, Flexi tray, Natta float bubble tray, ballast tray, cascade tray, venturi tray, Kitter tray, recycling tray, chimney tray, Z tray, turbo grid tray, ripple tray, dual flow tray or baffle tray. If a packed tower is used, the packing can be of any type such as a ring type, saddle type, Dixon ring, Magmahon packing, spray pack, Pana pack, Goodroy packing, Stedman packing or cross spiral packing.

A distillation tower with a condenser installed at the top for partially refluxing the distillate can also be used without any problem.

The pressure of the second distillation tower of step (2) can be any of atmospheric pressure, higher pressure than atmospheric pressure or reduced pressure. It is preferable that the operation temperature at the tower bottom producing a lowermost station of the tower is near the boiling point of water at the pressure concerned, and that the operation temperature at the tower top producing the uppermost station of the tower is similarly near the azeotropic point of the cyclic formal and water at the pressure concerned.

In step (3), to further remove water from the azeotropic mixture consisting of the cyclic formal and water obtained in step (2), the azeotropic mixture consisting of the cyclic formal and water is supplied to a reflux portion or upper station of the azeotropic distillation tower, and separately, a hydrocarbon component, to form another azeotropic mixture with water, is supplied to a middle or higher station of the azeotropic distillation tower, for distillation. Thus, from a lower station of the azeotropic distillation tower, a purified cyclic formal can be obtained.

It is important that the hydrocarbon component used here has an azeotropic temperature with water of not higher than the boiling point of the cyclic formal, and, specifically, can be, for example, cyclopentane, cyclohexene or benzene. An especially preferable hydrocarbon component is benzene in view of its separation efficiency between the cyclic formal and water.

As the hydrocarbon component used to form an azeotropic mixture with water, the hydrocarbon component obtained by condensing by y a condenser or the hydrocarbon component vapor containing water, distilled from the top of the azeotropic distillation tower, and separating from the water phase of the condensate by a liquid-liquid separator can be refluxed to the azeotropic distillation tower.

Since the rectification in step (3) is intended to separate water, the condensed and separated water phase is of course discharged outside the azeotropic distillation tower. In this case, if the water phase discharged outside the azeotropic distillation tower is at least partially returned to the azeotropic distillation tower, the rectification efficiency can be remarkably improved.

As for the quantity of the distillate to be refluxed, it is only required that the molar ratio of the total quantity of the hydrocarbon component used in circulation in the azeotropic distillation tower and newly supplied to it, to the total quantity of water used in circulation in the azeotropic distillation tower and newly supplied to it is kept preferably at 0.8 to 1.2. As a result, while the water content of the residue from the azeotropic distillation tower is kept sufficiently low, the accompanying hydrocarbon component can be stably controlled.

It is preferable that the distillate is refluxed to a station higher than the station to which the hydrocarbon component to form an azeotropic mixture with water is supplied, or that both are supplied to the reflux portion. As a result, the azeotropic composition can be easily formed, so as to prevent the water re-supplied as the distillate separated from the azeotropic distillation tower from being present in the residue.

In the present invention, it is preferable that the added amount of the hydrocarbon component to form an azeotropic mixture with water is 0.1 to 10 as a ratio by weight to the amount of the azeotropic mixture consisting of the cyclic formal and water. A more preferable range is 0.5 to 5. Since the hydrocarbon component accompanies the distillate coming from the top of the azeotropic distillation tower and the residue, the amount of the hydrocarbon component to be newly supplied from outside corresponds to the amount to make up for the shortage in the distillation tower. The amount of the hydrocarbon component to be newly supplied from outside is usually 0.001 to 1 as a ratio by weight to the amount of the azeotropic mixture consisting of the cyclic formal and water.

The pressure of the azeotropic distillation tower for purifying the cyclic formal is any of atmospheric pressure, higher pressure than atmospheric pressure or reduced pressure. It is preferable that the operation temperature at the tower bottom is about the boiling point of the cyclic formal at the pressure concerned, and that the operation temperature at the tower top is similarly about the azeotropic point of the hydrocarbon component, cyclic formal and water at the pressure concerned.

The azeotropic distillation tower used for purification in step (3) may be any ordinary known distillation tower such as that used for the second distillation tower. If a plate tower is used, the plate type can be any type such as a bubble cap, sieve tray, Uniflux tray, Flexi tray, Natta float bubble tray, ballast tray, cascade tray, venturi tray, Kitter tray, recycling tray, chimney tray, Z tray, turbo grid tray, ripple tray, dual flow tray or baffle tray. If a packed tower is used, the packing can be of any type such as a ring type, saddle type, Dixon ring, Magmahon packing, spray pack, Pana pack, Goodroy packing, Stedman packing or cross spiral packing.

In the method of the present invention for continuously producing a purified cyclic formal, raw formaldehyde is supplied to the reactor of a reaction distillation tower, and an alkylene glycol is supplied to the distillation tower of the reaction distillation tower, to produce a cyclic formal with a small unreactive formaldehyde content. Furthermore, the cyclic formal with a small unreactive formaldehyde content is distilled, and rectified with a hydrocarbon solvent to form an azeotropic mixture with water. Thus, a cyclic formal with a small water content can be easily produced at a high yield. The method for continuously producing a cyclic formal of the present invention is economical and can be simplified in operation industrially very significantly.

Preferred embodiments of the invention will now be described in more detail with reference to the accompanying drawings and Examples. In the drawings:

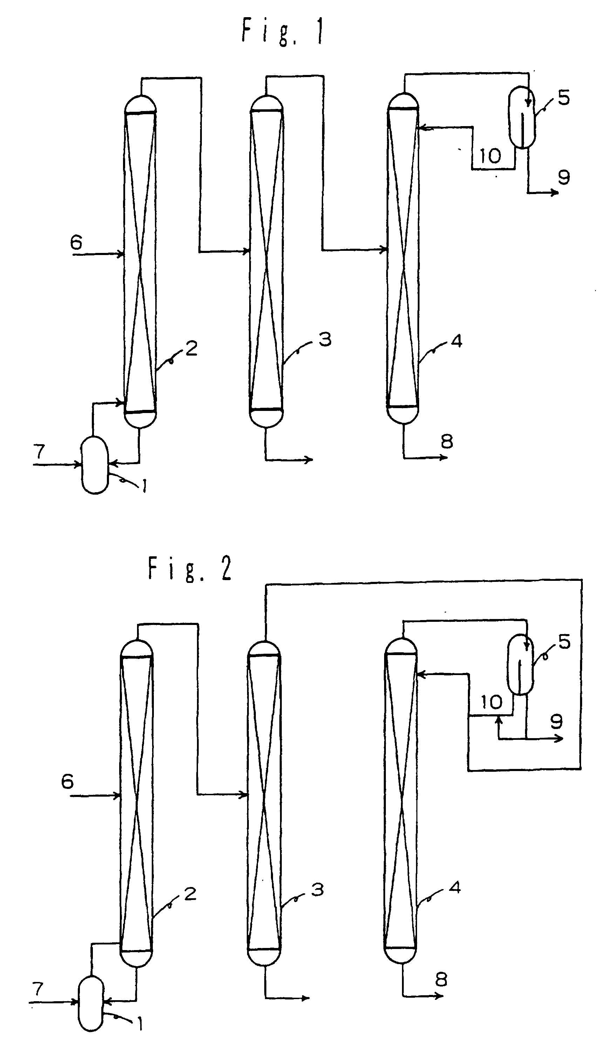

Fig. 1 is a schematic process drawing for illustrating a cyclic formal production process embodying the present invention.
Fig. 2 is a schematic process drawing for illustrating another cyclic formal production process embodying the present invention.

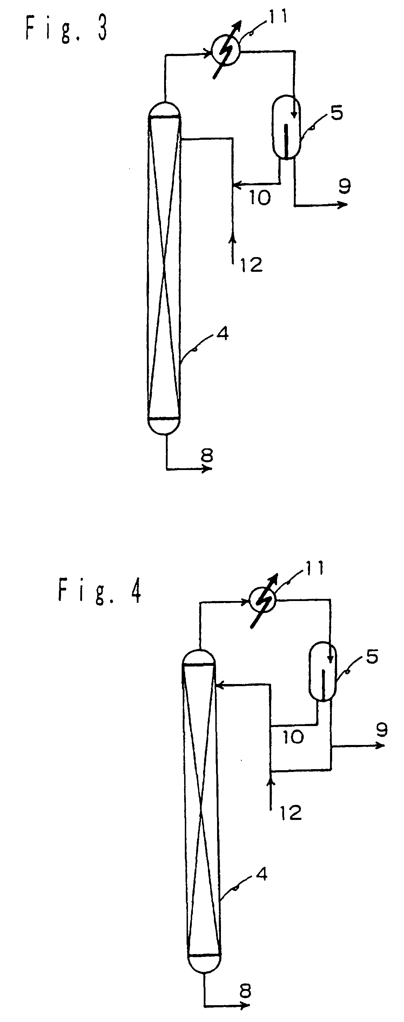

Fig. 3 is a schematic process drawing for illustrating a further, purification step, of a process embodying the present invention.
Fig. 4 is a schematic process drawing for illustrating a still further, purification steps of a process embodying the present invention.

In Fig. 1, a reaction distillation tower (not numbered) consists of a reactor 1 and a distillation tower 2. As indicated by arrows, the reactor 1 is connected with the distillation tower 2, and an alkylene glycol is supplied from the bottom of the distillation tower 2 to the reactor 1. Furthermore, from the reactor 1, a product and an unreacted mixture are supplied to the distillation tower 2.

In the present invention, at first, predetermined amounts of an alkylene glycol, formalin and catalyst are initially supplied into the reactor 1, and the temperature in the reactor 1 is heated to a predetermined level.

Then, formalin is continuously supplied from a formalin supply portion 7 into the reactor 1, and the alkylene glycol is continuously supplied from an alkylene glycol supply portion 6 into the distillation tower 2.

The alkylene glycol supplied into the distillation tower 2 contacts the unreacted formaldehyde coming from the reactor 1 in counter current, to absorb or react with it, while entering the reactor 1. On the other hand, formaldehyde is directly supplied into the reactor 1, to let unreacted formaldehyde contact the alkylene glycol in counter current. In this case, for better counter current contact of both, it is preferable that the alkylene glycol is added to a middle or higher station of the distillation tower 2. Thus, a distillate mainly consisting of the alkylene glycol formal and water and small in formaldehyde content (reaction distillate) can be obtained. Furthermore, at the top of the distillation tower 2, a condenser (not illustrated) can also be installed to partially reflux the distillate.

Subsequently, the distillate is supplied to a second distillation tower 3 for continuous distillation. From the bottom of the second distillation tower 3, water is taken out, and from the tower top, an azeotropic mixture consisting of the alkylene glycol formal and water can be obtained.

The azeotropic mixture is continuously supplied to an azeotropic distillation tower 4 for purification shown in Fig. 1, and separately, a hydrocarbon compound is continuously supplied to an upper station of the azeotropic distillation tower 4, for distillation in the azeotropic distillation tower 4.

In this case, a vapor is taken out from the top of the azeotropic distillation tower 4. The taken out vapor is condensed by a condenser (not illustrated), and separated into hydrocarbon compound phase and water phase in a liquid-liquid separator 5. The hydrocarbon compound is continuously supplied to an upper station of the azeotropic distillation tower 4 through a reflux pipe 10. From the bottom of the liquid-liquid separator 5, distillation drain 9 is taken out. From the residue portion 8 at the bottom of the azeotropic distillation tower 4, the purified alkylene glycol formal is taken out.

Fig. 2 shows another embodiment slightly different from Fig. 1. The azeotropic mixture obtained in the second distillation tower of Fig. 1 is continuously supplied to the reflux line of the azeotropic distillation tower 4 for purification shown in Fig. 2. Separately, the hydrocarbon compound is also continuously supplied through the reflux line to the azeotropic distillation tower 4. The vapor taken out of the top of the azeotropic distillation tower 4 is condensed by a condenser (not illustrated) and separated into hydrocarbon compound phase and water phase in the liquid-liquid separator 5. The obtained hydrocarbon compound is continuously supplied through the reflux pipe 10 to an upper station of the azeotropic distillation tower 4.

From the bottom of the liquid-liquid separator 5, distillation drain 9 is taken out. The condensed and separated water phase is of course discharged outside the system, but as illustrated, the water phase discharged outside the system is at least partially returned to the azeotropic distillation tower 4. Thus, the rectification efficiency can be significantly improved. From the residue portion 8 at the bottom of the azeotropic distillation tower 4, the purified alkylene glycol formal is taken out.

Fig. 3 shows a further other embodiment of the azeotropic distillation tower 4.

The azeotropic mixture consisting of the alkylene glycol formal and water is continuously supplied to the reflux line of the azeotropic distillation tower 4 shown in Fig. 3, and separately the hydrocarbon compound is also continuously supplied through the reflux line to the azeotropic distillation tower 4. Subsequently, the vapor taken out of the top of the azeotropic distillation tower 4 is condensed by a condenser 11, and separated into hydrocarbon compound phase and water phase in the liquid-liquid separator 5. The obtained hydrocarbon compound is continuously supplied to the an upper station of the azeotropic distillation tower 4 through reflux pipe 10.

In this case, to make up for the hydrocarbon compound decreased in the azeotropic distillation tower 4, the hydrocarbon compound can be additionally supplied through the reflux pipe 10 to a hydrocarbon compound supply port 12. From the residue portion 8 at the bottom of the azeotropic distillation tower 4, the purified alkylene glycol formal is taken out.

Fig. 4 shows a still further other embodiment of the azeotropic distillation tower 4. The azeotropic mixture consisting of the alkylene glycol formal and water is continuously supplied to the azeotropic distillation tower 4 through the reflux line of the azeotropic distillation tower 4 shown in Fig. 4. Furthermore, the hydrocarbon compound is also continuously supplied to the azeotropic distillation tower 4 through the reflux line. Subsequently, the vapor taken out of the top of the azeotropic distillation tower 4 is condensed by the condenser 11, and separated into hydrocarbon compound phase and water phase in the liquid-liquid separator 5. The hydrocarbon compound obtained in this manner is continuously supplied an upper station of the azeotropic distillation tower through the reflux pipe 10. In this case, to make up for the hydrocarbon compound decreased in the azeotropic distillation tower 4, the hydrocarbon compound can be additionally supplied through the reflux pipe 10 to the hydrocarbon compound supply port 12. Furthermore, the water phase condensed and separated in the liquid-liquid separator 5 is discharged outside the system, but as illustrated, the water phase can be returned at least partially to the azeotropic distillation tower 4, for significantly improve the rectification efficiency. From the residue portion 8 at the bottom of the azeotropic distillation tower 4, the purified alkylene glycol formal is taken out.

### Examples

### Example 1

In this Example, a reaction distillation tower consisting of a reactor 1 (with a capacity of 800 ml) and a distillation tower 2 (Older Shaw plate tower with a diameter of 30 mm and 20 sieve trays installed) was used.

At first, 270 g (4.4 moles) of ethylene glycol, 130 g of 60% formalin (2.6 moles of formaldehyde) and 4 g of sulfuric acid were initially supplied into the reactor 1, and the reactor 1 was heated until the inside temperature reached 120°C. When the inside temperature reached 120°C, 60% formalin was continuously supplied to the reactor 1 from the formalin supply port 7 at a rate of 85 g/hr (1.7 moles of formaldehyde per hour). Separately, ethylene glycol was continuously supplied from the hydrocarbon compound supply port 6 at the 10th station from the bottom of the distillation tower 2 at a flow rate of 106 g/hr (1.7 moles/hr).

The ethylene glycol supplied to the distillation tower was introduced into the reactor I while being brought into contact with the unreactive formaldehyde coming from the reactor in counter current, for reaction with formaldehyde.

In the reaction distillation tower, it took 120 minutes to obtain a distillate with a stable composition (reaction distillate). The distillate was analyzed by gas chromatography and found to contain 65.4 wt% of ethylene glycol formal, 33.7 wt% of water and 0. 1 wt% of formaldehyde.

### Example 2

Ethylene glycol formal was produced as described in Example 1, except that the amounts of ethylene glycol and 60% formalin initially supplied into the reactor 1 were 140 g (2.3 moles) of ethylene glycol and 200 g of 60% formalin (4 moles of formaldehyde). The obtained distillate (reaction distillate) was analyzed by gas chromatography and found to contain 62.8 wt% of ethylene glycol formal, 33. 1 wt% of water and 1.0 wt% of formaldehyde.

It can be seen that if the molar ratio of ethylene glycol to formaldehyde in the reactor 1 is larger than 1, the amount of formaldehyde as impurity in ethylene glycol formal is obviously small.

### Example 3

A reaction distillation tower of the same type as that of Example 1 except that the distillation tower 2 was a packed tower with a diameter of 30 mm, two distillation station and ring type packing.

Two hundred and seventy grams (4.4 moles) of ethylene glycol, 130 g of 60% formalin (2.6 moles of formaldehyde) and 4 g of sulfuric acid were initially supplied into the reactor, and the reactor was heated till the inside temperature reached 110°C. When the inside temperature of the reactor 1 reached 120°C, 60% formalin was continuously supplied into the reactor 1 at a flow rate of 85 g/hr (1.7 moles of formaldehyde per hour), and ethylene glycol was continuously supplied to the iststationfrom the bottom of the distillation tower 2 at a flow rate of 106 g/hr (1.7 moles/hr).

In the reaction distribution tower, it took 120 minutes to obtain a distillate with a stable composition (reaction distillate). The distillate was analyzed by gas chromatography and found to contain 64.3 wt% of ethylene glycol formal, 33.4 wt% of water and 0.5 wt% of formaldehyde.

### Example 4

A reaction distillation tower consisting of a reactor 1 (with a capacity of 800 ml) and a distillation tower (Older Shaw plate tower with a diameter of 30 mm and 20 sieve trays installed) shown in Fig. 1 was used.

Two hundred and seventy grams (4.4 moles) of ethylene glycol, 130 g of 60% formalin (2.6 moles of formaldehyde) and 4 g of sulfuric acid were initially supplied into the reactor 1, and the reactor 1 was heated till the inside temperature reached 120°C. When the inside temperature of the reactor 1 reached 120°C, 60% formalin was continuously supplied from the formalin supply port 7 into the reactor 1 at a flow rate of 85 g/hr (1.7 moles of formaldehyde per hour), and ethylene glycol was continuously supplied from the ethylene glycol supply port 6 at the 10th station from the bottom of the distillation tower 2 at a flow rate of 106 g/hr (1.7 moles/hr).

It took 120 minutes to obtain a distillate with a stable composition (reaction distillate). The distillate was analyzed by gas chromatography and found to contain 65.4 wt% of ethylene glycol formal, 33.7 wt% of water and 0. 1 wt% of formaldehyde.

In succession, the distillate was supplied into the distillation tower 3 (plate tower with a diameter of 30 mm and 20 sieve trays) at the 10th station from the bottom of the distillation tower at a flow rate of 191 g/hr, for continuous distillation at a tower bottom temperature of 103°C. It took 120 minutes to obtain a distillate with a stable composition. From the top of the second distillation tower 3, an azeotropic mixture containing 91.8 wt% of ethylene glycol formal and 7.6 wt% of water was obtained.

The azeotropic mixture was continuously supplied to the 10th station from the bottom of the azeotropic distillation tower 4 (plate tower with a diameter of 30 mm and 40 sieve trays) for purification shown in Fig. 1 at a flow rate of 136 /hr, and benzene was continuously supplied to the 3rd station from the top of the azeotropic distillation tower 4 at a flow rate of 16 g/hr. The distillation operation in the azeotropic distillation tower 4 was conducted at atmospheric pressure at a tower bottom temperature of 77°C. Subsequently, the vapor from the tower top was condensed and separated into benzene phase and water phase in the liquid-liquid separator 5, and the benzene phase was continuously supplied to the 1st station from the top of the azeotropic distillation tower 4 at a flow rate of 367 g/hr.

From the residue portion 8 at the tower bottom, purified ethylene glycol formal containing 11 wt% of benzene was obtained at 135 g/hr. The yield of ethylene glycol formal to the initially supplied ethylene glycol and formaldehyde was 97.5%. The water content was measured according to the Karl Fischer method and found to be 44 ppm.

### Example 5

The azeotropic mixture obtained in Example 4 (containing 91.8 wt% of ethylene glycol formal and 7.6 wt% of water) was continuously supplied into the reflux line of the azeotropic distillation tower 4 (plate tower with a diameter of 30 mm and 40 sieve trays) for purification shown in Fig. 2 at a flow rate of 136 g/hr, and benzene was also continuously supplied into the reflux line at a flow rate of 16 g/hr. The azeotropic distillation tower 4 was operated at atmospheric pressure at a tower bottom temperature of 77°C. Subsequently, the vapor taken out of the top of the azeotropic distillation tower 4 was condensed and separated into benzene phase and water phase in the liquid-liquid separator 5, and the benzene was continuously supplied to the 1st station from the tower top at a flow rate of 367 g/hr. Furthermore, supply of water was gradually started, and finally water was continuously supplied to the top of the azeotropic distillation tower 4 at a flow rate of 9 g/hr. From the residue portion 8 at the tower bottom, purified ethylene glycol formal containing 0.7 wt% of benzene was obtained at 135 g/hr. The yield of ethylene glycol formal to the initially supplied ethylene glycol and formaldehyde was 97.5%. The water content was measured according the Karl Fischer method and found to be 22 ppm.

### Comparative Example 1

Ethylene glycol formal was produced as described in Example 4, except that ethylene glycol was supplied together with formaldehyde into the reactor, instead of supplying ethylene glycol to the 10th station from the bottom of the distillation tower 2. It was analyzed as described in Example 4 and found to contain 63.4 wt% of ethylene glycol formal, 33.8 wt% of water and 1.8 wt% of formaldehyde. It can be seen that the product of the present invention is obviously smaller in the content of aldehyde as impurity in ethylene glycol formal.

The distillate was purified by the azeotropic distillation tower 4 after distillation in the second distillation tower as described in Example 4. The yield of ethylene glycol formal was 91.4%, and the water content measured according to the Karl Fischer method was 46 ppm.

### Comparative Example 2

Ethylene glycol formal was obtained as described in Example 1, except that methanol was added to the azeotropic distillation tower 4 instead of benzene. In this case, the yield of ethylene glycol formal was 97.0%. The water content was measured by gas chromatography and found to be 6.5 wt%.

### Example 6

A reaction distillation tower consisting of a reactor with a capacity of 800 ml and a distillation tower 2 (plate tower with a diameter of 30 mm and 20 sieve trays) shown in Fig. 1 was used.

Two hundred and seventy grams (4.4 moles) of ethylene glycol, 130 g of 60% formalin (2.6 moles of formaldehyde) and 4 g of sulfuric acid were initially supplied into the reactor, and the reactor 1 was heated till the inside temperature reached 120°C. When the inside temperature of the reactor 1 reached 120°C, 60% formalin was continuously supplied into the reactor 1 at a flow rate of 85 g/hr (1.7 moles of formaldehyde per hour), and ethylene glycol was continuously supplied into the distillation tower 2 at a flow rate of 106 g/hr (1.7 moles/hr), for operation at a reflux ratio of 0.5. It took 120 minutes to obtain a distillate with a stable composition (reaction distillate). The distillate was analyzed by gas chromatography and found to contain 65.4 wt% of ethylene glycol, 33.7 wt% of water and 0. 1 wt% of formaldehyde. The distillate was supplied into the second distillation tower 3 (plate tower with a diameter of 30 mm and 20 sieve trays), heated to 74°C at atmospheric pressure, and distilled at a reflux ratio of 0.6, to obtain an azeotropic mixture containing 91.8 wt% of ethylene glycol formal and 7.6 wt% of water from the top of the second distillation tower.

The azeotropic mixture containing ethylene glycol formal and water was continuously supplied to the 10th station from the bottom of the azeotropic distillation tower (plate tower with diameter of 30 mm and 40 sieve trays) shown in Fig. 1 at a flow rate of 211 g/hr, and benzene was continuously supplied to the 3rd station from the tower top at a flow rate of 24 g/hr, for operation at atmospheric pressure at a tower bottom temperature of 77°C. Then, the vapor from the tower top was condensed and separated into benzene phase and water phase in the liquid-liquid separator, and the benzene phase was continuously supplied to the tower top at a flow rate of 570 g/hr. Purified ethylene glycol formal was obtained from the tower bottom. When measured according to the Karl Fischer method, the water content was found to be 40 ppm and the benzene content was found to be 12 wt%.

### Example 7

The azeotropic mixture containing 91.8 wt% of ethylene glycol formal and 7.6 wt% of water obtained in Example 6 was continuously supplied into the reflux line of the azeotropic distillation tower 4 (plate tower with a diameter of 30 mm and 40 sieve trays) shown in Fig. 3 at a flow rate of 211 g/hr, and benzene was also continuously supplied to the reflux line at a flow rate of 24 g/hr, for operation at atmospheric pressure at a tower bottom temperature of 77°C. Subsequently, the vapor from the top of the azeotropic distillation tower 4 was condensed and separated into benzene phase and water phase in the liquid-liquid separator 5, and the benzene phase was continuously supplied to the top of the azeotropic distillation tower 4 at a flow rate of 570 g/hr. From the bottom of the azeotropic distillation tower 4, purified ethylene glycol formal was obtained. When measured according to the Karl Fischer method, the water content was found to be 20 ppm and the benzene content was found to be 12 wt% as before.

### Example 8

The azeotropic mixture containing 91.8 wt% of ethylene glycol formal and 7.6 wt% of water obtained in Example 6 was continuously supplied into the reflux line of the azeotropic distillation tower 4 (plate tower with a diameter of 30 mm and 40 sieve trays) shown in Fig. 4 at a flow rate of 211 g/hr, and benzene was also continuously supplied into the reflux line at a flow rate of 24 g/hr, for operation at atmospheric pressure at a tower bottom temperature of 77°C. Subsequently, the vapor from the tower top was condensed by the condenser 11 and separated into benzene phase and water phase in the liquid-liquid separator 5, and the benzene phase was continuously supplied to the top of the azeotropic distillation tower 4 at a flow rate of 570 g/hr. Furthermore, supply of water phase was gradually started, and finally water was continuously supplied to the tower top at a flow rate of 14 g/hr. When the distillate and the residue of the azeotropic distillation tower 4 became stable in composition, purified ethylene glycol formal was obtained from the bottom of the azeotropic distillation tower 4. When measured according to the Karl Fischer method, the water content was found to be 20 ppm and the benzene content was found to be 0.8 wt%.

## Claims

1. A method for continuously producing a cyclic formal, in which a reaction distillation tower equipped with a reactor (1) and a distillation tower (2) is used for letting an alkylene glycol component and a formaldehyde component react with each other in the presence of a catalyst, to produce a cyclic formal, comprising the step of continuously supplying the alkylene glycol component from the distillation tower (2) to the reactor (1) of the reaction distillation tower, while continuously supplying formaldehyde component to the reactor (1) of the reaction distillation tower, for letting the alkylene glycol and formaldehyde components react with each other.

2. A method according to claim 1, wherein the alkylene glycol component supplied to the distillation tower is brought into contact with unreacted formaldehyde component coming from the reactor (1) in counter current, to absorb or react with the formaldehyde component, while being introduced into the reactor (1), for reaction with the formaldehyde component.

3. A method according to claim 1 or claim 2, wherein the molar ratio of the alkylene glycol component to formaldehyde component in the reactor is kept at 1 to 100, while the amounts of the alkylene glycol component and the formaldehyde component continuously supplied into the distillation tower (2) and the reactor (1) are equimolar.

4. A method according to any preceding claim, wherein the alkylene glycol component is at least one alkylene glycol selected from ethylene glycol, diethylene glycol and 1,4-butanediol.

5. A method according to any preceding claim, wherein the reaction distillation tower (2) used has at least two distillation stations in the distillation tower (2).

6. A method according to any preceding claim, wherein the distillation tower (2) used in the reaction distillation tower is a plate tower and/or a packed tower.

7. A method according to any one of claims 1 to 6, which comprises
(1) the step of continuously supplying the alkylene glycol component from the distillation tower (2) to the reactor (1) of the reaction distillation tower while continuously supplying the formaldehyde component to the reactor (1) of the reaction distillation tower, for letting the alkylene glycol and formaldehyde components react with each other, to continuously obtain a reaction distillate containing water and a cyclic formal from an upper station of the distillation tower,
(2) the step of continuously supplying the reaction distillate containing water and a cyclic formal obtained in step (1) to a second distillation tower (3), to continuously obtain an azeotropic mixture consisting of the cyclic formal and water, and
(3) the step of supplying the azeotropic mixture consisting of the cyclic formal and water obtained in step (2) to an azeotropic distillation tower (4), while supplying a hydrocarbon component, the azeotropic point of which with water is not higher than the boiling point of the cyclic formal, for continuous distillation, and obtaining the azeotropic mixture consisting of the hydrocarbon component and water from an upper station of the azeotropic distillation tower (4) while continuously obtaining the cyclic formal purified after removing water, from a lower station of the azeotropic distillation tower (4).

8. A method according to claim 7, wherein in step (3), the hydrocarbon component is at least one hydrocarbon compound selected from cyclopentane, cyclohexene and benzene.

9. A method according to claim 7 or 8, wherein in step (3), the azeotropic mixture consisting of a cyclic formal and water is supplied to a reflux portion or upper station of the azeotropic distillation tower (4).

10. A method according to any one of claims 7 to 9, wherein in the step (3), the distillate from the azeotropic distillation tower (4) is refluxed at least partially to the azeotropic distillation tower, without discharging all of the quantity of the distillate outside the system.

11. A method according to claim 1, which method further comprises continuously purifying the cyclic formal, by removal of water by distillation from an azeotropic mixture of the cyclic formal and water, the purification method comprising the step of adding a hydrocarbon component, which is at least one hydrocarbon solvent selected from cyclopentane, cyclohexane and benzene and the azeotropic point of which component with water is not higher than the boiling point of the cyclic formal, to the cyclic formal containing water, for continuous distillation.

12. A method according to claim 11, wherein the cyclic formal is at least one formal selected from a group consisting of ethylene glycol formal, diethylene glycol formal and 1,4-butanediol formal.

13. A method according to claim 11 or 12, wherein the cyclic formal containing water is supplied to a reflux portion or upper station of a distillation tower (3 or 4).

14. A method according to any one of claims 11 to 13, wherein the distillate from the distillation tower (3 or 4) is refluxed at least partially to the distillation tower (3 or 4), without discharging all of the quantity of the distillate outside the system.

## Patentansprüche

1. Verfahren zur kontinuierlichen Herstellung eines zyklischen Formals, bei dem eine Reaktionsdestillationskolonne, die mit einem Reaktor (1) und einer Destillationskolonne (2) ausgestattet ist, verwendet wird, um eine Alkylenglykol-Komponente und eine Formaldehyd-Komponente in Gegenwart eines Katalysators miteinander reagieren zu lassen, um ein zyklisches Formal zu erzeugen, umfassend den Schritt des kontinuierlichen Zuführens der Alkylenglykol-Komponente von der Destillationskolonne (2) zum Reaktor (1) der Reaktionsdestillationskolonne, während dem Reaktor (1) der Reaktionsdestillationskolonne kontinuierlich Formaldehyd zugeführt wird, um die Alkylenglykolund die Formaldehyd-Komponente miteinander reagieren zu lassen.

2. Verfahren nach Anspruch 1, worin die der Destillationskolonne zugeführte Alkylenglykol-Komponente mit nichtumgesetzter Formaldehyd-Komponente in Kontakt gebracht wird, die im Gegenstrom vom Reaktor (1) her kommt, um die Formaldehyd-Komponente zu absorbieren oder damit zu reagieren, während sie in den Reaktor (1) eingeleitet wird, um mit der Formaldehyd-Komponente zu reagieren.

3. Verfahren nach Anspruch 1 oder 2, worin das Molverhältnis zwischen der Alkylenglykol-Komponente und der Formaldehyd-Komponente im Reaktor bei 1 bis 100 gehalten wird, während die Mengen der Alkylenglykol-Komponente und der Formaldehyd-Komponente, die der Destillationskolonne (2) und dem Reaktor (1) kontinuierlich zugeführt werden, äquimolar sind.

4. Verfahren nach einem der vorangegangenen Ansprüche, worin die Alkylenglykol-Komponente zumindest eine aus Ethylenglykol, Diethylenglykol und 1,4-Butandiol ausgewählte Alkylenglykol-Komponente ist.

5. Verfahren nach einem der vorangegangenen Ansprüche, worin die eingesetzte Reaktionsdestillationskolonne (2) zumindest zwei Destillationsstationen in der Destillationskolonne (2) aufweist.

6. Verfahren nach einem der vorangegangenen Ansprüche, worin die in der Reaktionsdestillationskolonne eingesetzte Destillationskolonne (2) eine Bodenkolonne und/oder eine gepackte Kolonne ist.

7. Verfahren nach einem der Ansprüche 1 bis 6, das Folgendes umfasst:
(1) den Schritt des kontinuierlichen Zuführens der Alkylenglykol-Komponente von der Destillationskolonne (2) zum Reaktor (1) der Reaktionsdestillationskolonne, während die Formaldehyd-Komponente kontinuierlich dem Reaktor (1) der Reaktionsdestillationskolonne zugeführt wird, um die Alkylenglykol- und die Formaldehyd-Komponente miteinander reagieren zu lassen, um kontinuierlich von einer oberen Station der Destillationskolonne ein Reaktionsdestillat zu erhalten, das Wasser und ein zyklisches Formal enthält,
(2) den Schritt des kontinuierlichen Zuführens des in Schritt (1) erhaltenen Reaktionsdestillats, das Wasser und ein zyklisches Formal enthält, zu einer zweiten Destillationskolonne (3), um kontinuierlich ein azeotropes Gemisch zu erhalten, das aus dem zyklischen Formal und Wasser besteht, und
(3) den Schritt des Zuführens des in Schritt (2) erhaltenen azeotropen Gemisches, das aus dem zyklischen Formal und Wasser besteht, zu einer Azeotrop-Destillationskolonne (4), während eine Kohlenwasserstoff-Komponente, deren Azeotroppunkt mit Wasser nicht höher als der Siedepunkt des zyklischen Formals liegt, zur kontinuierlichen Destillation zugeführt wird, und des Erhaltens des azeotropen Gemisches, das aus der Kohlenwasserstoff-Komponente und Wasser besteht, von einer oberen Station der Azeotrop-Destillationskolonne (4), während kontinuierlich von einer unteren Station der Azeotrop-Destillationskolonne (4) das nach dem Entfernen von Wasser gereinigte zyklische Formal erhalten wird.

8. Verfahren nach Anspruch 7, worin die Kohlenwasserstoff-Komponente in Schritt (3) zumindest eine aus Cyclopentan, Cyclohexen und Benzol ausgewählte Kohlenwasserstoffverbindung ist.

9. Verfahren nach Anspruch 7 oder 8, worin das aus einem zyklischen Formal und Wasser bestehende azeotrope Gemisch in Schritt (3) einem Rückflussabschnitt oder einer oberen Station der Azeotrop-Destillationskolonne (4) zugeführt wird.

10. Verfahren nach einem der Ansprüche 7 bis 9, worin das Destillat aus der Azeotrop-Destillationskolonne (4) in Schritt (3) zumindest teilweise zur Azeotrop-Destillationskolonne zurückfließen gelassen wird, ohne die gesamte Menge des Destillats aus dem System abzugeben.

11. Verfahren nach Anspruch 1, wobei das Verfahren weiters das kontinuierliche Reinigen des zyklischen Formals durch Entfernen von Wasser mittels Destillation aus einem azeotropen Gemisch aus dem zyklischen Formal und Wasser umfasst, wobei das Reinigungsverfahren den Schritt des Hinzufügens einer Kohlenwasserstoff-Komponente, die zumindest ein aus Cyclopentan, Cyclohexan und Benzol ausgewähltes Kohlenwasserstoff-Lösungsmittel ist, wobei der Azeotroppunkt der Komponente mit Wasser nicht höher als der Siedepunkt des zyklischen Formals liegt, zum Wasser enthaltenden zyklischen Formal zur kontinuierlichen Destillation umfasst.

12. Verfahren nach Anspruch 11, worin das zyklische Formal zumindest ein aus der aus Ethylenglykolformal, Diethylenglykolformal und 1,4-Butandiolformal bestehenden Gruppe ausgewähltes Formal ist.

13. Verfahren nach Anspruch 11 oder 12, worin das Wasser enthaltende zyklische Formal einem Rückflussabschnitt oder einer oberen Station einer Destillationskolonne (3 oder 4) zugeführt wird.

14. Verfahren nach einem der Ansprüche 11 bis 13, worin das Destillat aus der Destillationskolonne (3 oder 4) zumindest teilweise zur Destillationskolonne (3 oder 4) zurückfließen gelassen wird, ohne die gesamte Menge des Destillats aus dem System abzugeben.

## Revendications

1. Un procédé pour la préparation d'un formal cyclique, dans lequel une tour de distillation réactionnelle équipée d'un réacteur (1) et d'une tour de distillation (2) est utilisée pour amener un composant d'alkylène glycol et un composant de formaldéhyde à réagir l'un avec l'autre en présence d'un catalyseur, afin de produire un formal cyclique, comprenant l'étape consistant à amener de manière continue le composant d'alkylène glycol depuis la tour de destination (2) jusqu'au réacteur (1) de la tour de distillation réactionnelle, tout en amenant de manière continue le composant de formaldéhyde vers le réacteur (1) de la tour de distillation réactionnelle, pour amener les composants d'alkylène glycol et de formaldéhyde à réagir l'un avec l'autre.

2. Un procédé selon la revendication 1, dans lequel le composant d'akylène glycol amené à la tour de distillation est mis en contact avec le composant de formaldéhyde non réagi provenant du réacteur (1) à contre courant, afin d'absorber ou de réagir avec le composant de formaldéhyde, tout en étant introduit dans la réacteur (1) en vue d'une réaction avec le composant de formaldéhyde.

3. Un procédé selon la revendication 1 ou la revendication 2, dans lequel le rapport molaire du composant d'alkylène glycol au composant de formaldéhyde dans la réacteur est maintenu de 1 à 100, alors que les quantités du composant d'alkylène glycol et du composant de formaldéhyde amené en continu dans la tour de distillation (2) et le réacteur (1) sont équimolaires.

4. Un procédé selon une quelconque revendication prédédente, dans lequel le composant d'alkylène glycol est au moins un alkylène glycol choisi parmi l'éthylène glycol, le diéthylène glycol et le 1,4-butanédiol.

5. Un procédé selon une quelconque revendication précédente, dans lequel la tour de distillation réactionnelle (2) utilisée présente au moins deux postes de distillation dans la tour de distillation (2).

6. Un procédé selon une quelconque revendication précédente, dans lequel la tour de distillation (2) utilisée dans la tour de distillation réactionnelle est une tour à plaque et/ou une tour garnie.

7. Un procédé selon une quelconque des revendications 1 à 6, qui comprend
(1) l'étape consistante à amener en continu le composant d'alkylène glycol depuis la tour de distillation (2) jusqu'au réacteur (1) de la tour de distillation réactionnelle tout en amenant de manière continue le composant de formaldéhyde vers la réacteur (1) de la tour de distillation réactionnelle, pour amener les composants d'alkylène glycol et de formaldéhyde à réagir l'un avec l'autre, afin d'obtenir de manière continue un distillat réactionnel contenant de l'eau et un formal cyclique à partir d'un poste supérieur de la tour de distillation,
(2) l'étape consistant à amener en continu le distillat réactionnel contenant de l'eau et un formal cyclique obtenu dans l'étape (1) vers une seconde tour de distillation (3), afin d'obtenir de manière continue un mélange azéotrope se composant du formal cyclique et de l'eau, et
(3) l'étape consistant à amener le mélange azéotrope se composant de formal cyclique et d'eau obtenu dans l'étape (2) vers une tour de distillation azéotrope (4), tout en amenant un composant hydrocarboné, dont le point azéotrope avec l'eau n'est pas supérieur au point d'ébullition du formal cyclique, en vue d'une distillation continue, et obtenir le mélange azéotrope se composant du composant hydrocarboné et d'eau à partir d'un poste supérieur de la tour de distillation azéotrope (4) tout en obtenant de manière continue le formal cyclique purifié après élimination de l'eau, à partir d'un poste inférieur de la tour de distillation azéotrope (4).

8. Un procédé selon la revendication 7, dans lequel, dans l'étape (3), le composant hydrocarboné est au moins un composé hydrocarboné choisi parmi le cyclopentane, le cyclohexène et le benzène.

9. Un procédé selon la revendication 7 ou 8, dans lequel, dans l'étape (3), le mélange azéotrope se composant d'un formal cyclique et d'eau est amené vers une partie de reflux ou un poste supérieur de la tour de distillation azéotrope (4).

10. Un procédé selon une quelconque des revendications 7 à 9, dans lequel, dans l'étape (3), le distillat provenant de la tour de distillation azéotrope (4) est renvoyée en reflux au moins partiellement vers la tour de distillation azéotrope, sans évacuer toute la quantité du distillat à l'extérieur du système.

11. Un procédé selon la revendication 1, procédé qui comprend en outre l'étape consistant à purifier de manière continue le formal cyclique, par un retrait de l'eau par distillation à partir d'un mélange azéotrope du formal cyclique avec de l'eau, le procédé de purification comprenant, en vue d'une distillation continue, l'étape consistant à ajouter au formal cyclique contenant de l'eau, un composant hydrocarboné, lequel est au moins un solvant hydrocarboné choisi parmi le cyclopentane, le cyclohexane et le benzène, composant dont le point azéotropope avec l'eau n'est pas supérieur au point d'ébullition du formal cyclique.

12. Un procédé selon la revendication 11, dans lequel le formal cyclique est au moins un formal choisi dans le groupe se composant du formal d'éthylène glycol, du formal de diéthylène glycol et du formal de 1,4-butanédiol.

13. Un procédé selon la revendication 11 ou 12, dans lequel le formal cyclique contenant de l'eau est amené à une portion de reflux ou à un poste supérieur d'une tour de distillation (3 ou 4).

14. Un procédé selon une quelconque des revendications 11 à 13, dans lequel le distillat provenant de la tour de distillation (3 ou 4) est renvoyée en reflux au moins partiellement vers la tour de distillation (3 ou 4), sans évacuer toute la quantité du distillat à l'extérieur du système.
